# NEUE EUROPÄISCHE PATENTSCHRIFT

(11) **EP 2 475 432 B2**
(45) Veröffentlichungstag und Bekanntmachung des Hinweises auf die Entscheidung über den Einspruch: **10.05.2023**
(45) Hinweis auf die Patenterteilung: 06.06.2018
(21) Anmeldenummer: 10745564.4
(22) Anmeldetag: 17.08.2010
(51) Int. Cl.: A61Q 15/00, A61K 8/34, A61K 8/49, A61K 8/37, A61Q 1/14, A61Q 17/00, A61Q 19/08, A61Q 19/00, A61K 8/92

(54) **KOSMETISCHE SPRAYFORMULIERUNG MIT ANTIMIKROBIELLER WIRKUNG**
Cosmetic spray formulation with antimicrobial effect
FORMULATION D'UN SPRAY COSMETIQUE DOTE D'UN EFFET ANTIMICROBIEN

(30) Priorität: 10.09.2009 EP 09011583
(43) Veröffentlichungstag der Anmeldung: 18.07.2012
(73) Patentinhaber: Dalli-Werke GmbH & Co. KG, 52224 Stolberg (DE)
(72) Erfinder: BONN, Andrea, 52372 Kreuzau (DE); ESSER, Christoph, 52146 Würselen (DE); GILLIAM, Herman, 6369 BH Simpelveld (NL); KRICHEL, Jürgen, 52428 Jülich (DE)
(74) Vertreter: f & e patent
(86) Internationale Anmeldenummer: PCT/EP2010/005049
(87) Internationale Veröffentlichungsnummer: WO 2011/029516

(56) Entgegenhaltungen:
- WO-A1-99/55349
- WO-A1-03/043593
- WO-A1-2008/080764
- WO-A2-03/018743
- WO-A2-2006/128622
- DE-A1- 4 111 569
- DE-A1- 4 125 560
- DE-U1-202004 021 418
- US-A- 5 082 652
- US-A- 5 989 531
- US-B1- 6 187 301
- US-B1- 6 231 842
- US-B1- 6 403 071
- anonymous: "Massageöle selber machen", , XP002568372, Gefunden im Internet: URL:http://www.waschkultur.de/massageoele- selbermachen.htm [gefunden am 2010-02-09]

## Beschreibung

Die vorliegende Erfindung betrifft eine Sprayformulierung, bestehend aus einem Gemisch einer kosmetischen Zusammensetzung und einem Gas oder Gasgemisch als Treibmittel, wobei das Gemisch aus kosmetischer Zusammensetzung und Gas als homogene flüssige Phase und Dampfdruckphase des Treibmittels vorliegt. Die kosmetische Zusammensetzung liegt in Form eines homogenen Gemisches vor, wobei das Gemisch neben einem antimikrobiellen Wirksystem einen besonders hohen Anteil an Öl-/Lipidphase aufweist.

Kosmetische Zusammensetzungen, die pflegende Substanzen und weitere Wirkstoffe enthalten, liegen in den häufigsten Fällen als Emulsionen, Dispersionen oder Suspensionen vor. Vor allem Artikel zur Hautpflege werden häufig als Öl-in-Wasser- (O/W) oder Wasser-in-Öl-Emulsionen (W/O) angeboten, wobei die verschiedenen pflegenden und eine andere Funktion erfüllenden Wirkstoffe in den unterschiedlichen Phasen gelöst oder dispergiert vorliegen können.

Körpergerüche entstehen durch die Einwirkung von Hautbakterien auf apokrinen Schweiß, wobei unangenehm riechende Abbauprodukte gebildet werden. Eine Gruppe der kosmetischen Zusammensetzungen mit Wirkstoffen, die nicht nur der Hautpflege, sondern vor allem einer anderen Funktion dienen, sind die Deodorants, bzw. die Antitranspirants. In diesen Zusammensetzungen sind entweder desodorierende Wirkstoffe enthalten, also Wirkstoffe, die einer Entwicklung von unangenehmen Körpergerüchen entgegenwirken sollen, oder Antitranspirantien, also Wirkstoffe, die die Bildung oder die Ausscheidung von Schweiß verringern adstringierende Wirkstoffe).

Üblicherweise werden Deodorants, die (einen) desodorierende(n) Wirkstoff(e) enthalten, in erster Linie als alkoholische Lösung, oder als Emulsion bereitgestellt. Solche Deodorants auf Alkoholbasis, wie sie bspw. in WO 03/043593 A1 beschrieben sind, haben allerdings den Nachteil, dass die Haut in den Bereichen, auf die das Deodorant aufgetragen wird, leicht austrocknet und es zu einer unangenehmen Hautreaktion wie Brennen oder Jucken kommen kann. Antitranspirants werden dagegen häufig als eine Dispersion eines antitranspiranten Wirkstoffes, üblicherweise eines Aluminiumsalzes, in einer Ölphase oder in Form einer O/W- oder einer W/O-Emulsion bereitgestellt. Bei solchen Zusammensetzungen kommt es in den meisten Fällen zur Bildung weißer Flecken oder Ränder auf Haut und Kleidung in dem Bereich, in dem die Zusammensetzung aufgetragen wurde, da das Salz sich nach Einziehen der Ölphase auf Haut und Kleidung niederschlägt. Antitranspirantzusammensetzungen sind bspw. in US 5,989,531, US 6,231,842 B1 und US 6,187,301 B1 beschrieben.

Deodorant- und Antitranspirantzusammensetzungen auf Basis von Silikonpolymeren sind aus US 6,403,071 B1 bekannt. Die dort beschriebenen Zusammensetzungen enthalten jedoch zwingend ein Hautpflegemittel in Form eines festen Salzes, welches erst bei Kontakt mit Achselschweiß oder anderer Unterarmfeuchtigkeit in Lösung geht und stellen somit keine homogenen Gemische dar.

Aufgabe der vorliegenden Erfindung war es eine kosmetische Zusammensetzung bereitzustellen, die der Entwicklung eines unangenehmen Körpergeruches durch Schwitzen entgegenwirkt, ein angenehmes Hautgefühl ergibt, die Haut nicht austrocknet und gute Pflegeeigenschaften aufweist. Darüber hinaus sollte die Bildung von weißen Rändern und Flecken auf der Haut und der Kleidung vermieden werden.
(a) Diese Aufgabe wird gelöst durch die Sprayformulierung gemäß Anspruch 1.

Die erfindungsgemäße Zusammensetzung zeichnet sich dadurch aus, dass sie beim Aufbringen auf die Haut eine hohe Pflegewirkung erzielt und der Entwicklung von unangenehmem Körpergeruch entgegenwirkt.

Ein weiteres Merkmal der Zusammensetzung ist es, dass sämtliche Bestandteile der Zusammensetzung miteinander mischbar sind oder ein Bestandteil in wenigstens einem der anderen Bestandteile lösbar ist, so dass sich insgesamt ein einphasiges, bevorzugt klares bis opaleszentes, homogenes Gemisch ergibt.

### Antimikrobielles Wirksystem (a):

Das antimikrobielle Wirksystem (a) umfasst mindestens einen Wirkstoff ausgewählt aus der Gruppe, enthaltend 2,4,4'-Trichlor-2'-hydroxydiphenylether (Triclosan), Phenoxyethanol, Ethylhexylglycerin, Parabenverbindungen, Isothiazoline und in organischen Lösungsmitteln lösliche Zinkverbindungen.

Die antimikrobiellen Substanzen sind in organischen Lösungsmitteln, insbesondere in Lösungsmitteln gemäß (b) oder (d) löslich, oder sie stellen selbst eine organische Flüssigkeit bzw. eine Flüssigkeit, die mit (b) oder (d) homogen mischbar ist, dar.

Parabenverbindungen können erfindungsgemäß eingesetzt werden. Parabenverbindungen schließen insbesondere ein: Methylparaben (CAS 99-76-3), Ethylparaben (CAS 120-47-8), Propylparaben (CAS 94-13-3), Butylparaben (CAS 94-26-8), Isopropylparaben (CAS 4191-73-5), and Benzylparaben (CAS 94-18-8). Parabene sind eine weitere gemäß der Erfindung bevorzugte Gruppe von antimikrobiellen Substanzen.

Ebenfalls wirksam gemäß der Erfindung einsetzbar sind Isothiazoline, insbesondere Methyl-4-isothiazolin, Benzisothiazolinon oder Benzchlorisothiazolinon, beispielsweise erhältlich unter dem Namen Acticide MBS der Firma Thor.

Eine weitere gemäß der Erfindung bevorzugte Gruppe von antimikrobiellen Substanzen sind in organischen Lösungsmitteln lösliche Zinkverbindungen, wie beispielsweise Zinkphenolsulfonat, Zinkoxide, Zinkchloride, Zinkundecylenat, und Mischungen davon.

Aus der Gruppe der antimikrobielle Wirkstoffe Ethylhexylglycerin, Phenoxyethanol, Wirkstoffe aus der Gruppe der Parabene oder der Isothiazoline, Triclosan, und den oben genannten Zinkverbindungen wird wenigstens ein Wirkstoff, bevorzugt eine Kombination von wenigstens zwei Wirkstoffen in die erfindungsgemäße Zusammensetzung eingearbeitet. Eine besonders bevorzugte Kombination ist Ethylhexylglycerin mit Phenoxyethanol, wobei zusätzlich ein weiterer Wirkstoff verwendet werden kann, aber nicht muß.

Das antimikrobielle Wirksystem stellt 20 bis 25 Gew% der gesamten kosmetischen Zusammensetzung dar. Das Wirksystem kann aus einer der oben genannten antimikrobiellen Substanzen bestehen, oder aus zwei, drei oder mehreren der Substanzen zusammengesetzt sein, wobei im letzteren Fall bevorzugt von jeder Substanz wenigstens 0,01 Gew% in der kosmetischen Zusammensetzung enthalten ist. Bei einer Kombination verschiedener Substanzen summieren sich deren Einsatzkonzentrationen auf den oben genannten Gesamtwert von maximal 25 Gew%.

Liegt eine der antimikrobiellen Substanzen in einem organischen Lösemittel gelöst vor, bezieht sich die oben genannte Einsatzkonzentration auf die reine Wirksubstanz, nicht auf das Lösemittel. Letzteres ist dem Lösungsmittelsystem (b) zuzuordnen.

### Lösemittelphase (b):

Das Lösemittelsystem (b) der erfindungsgemäßen Zusammensetzung dient in erster Linie der Lösung der Komponente(n) (a), soweit diese nicht selbst als Lösung in einem organischen Lösemittel, bevorzugt einem Lösemittel gemäß (b) oder in einem Öl oder Lipid gemäß (d) oder als organische Flüssigkeit vorliegen, sowie der Komponente(n) (c), um eine einphasige, also homogene Mischung aller Komponenten zu gewährleisten. Das Lösemittelsystem kann daher eine oder mehrere Komponenten umfassen, die wiederum selbst mit dem Öl-/ Lipidsystem (d) eine homogene Mischung ergeben. Diese Komponenten sind bevorzugt ausgewählt aus kurzkettigen Alkoholen mit 2 bis 6 Kohlenstoffatomen sowie deren Isomeren, wie Ethanol, Propanol, n-Butanol, Isobutanol, n-Pentanol, Isopentanol, n- Hexanol und Isohexanol, oder kurzkettigen Kohlenwasserstoffen mit 5 oder 6 Kohlenstoffatomen sowie deren Isomere, wie n-Pentan, Isopentan, n-Hexan oder Isohexan. Ebenso könnten Glycole, wie Monopropylenglycol, Dipropylenglycol und Glycolether, wie Dipropylenglykol-mono-methylether, 3-Butoxypropan-2-ol in dieser Komponente enthalten sein. Ein besonders bevorzugtes Lösemittel ist Isopentan. Ein weiteres bevorzugtes Lösemittel ist Ethanol.

Das Lösungsmittelsystem stellt 0 bis 70 Gew% der kosmetischen Zusammensetzung dar, bevorzugt 15 bis 60 Gew% und insbesondere bevorzugt 30 bis 50 Gew%. Im Falle, dass Ethanol als Lösemittel dient, ist der Einsatz dieser Komponente auf maximal 40 Gew% beschränkt, wobei das Lösungsmittelsystem neben dem Ethanol weitere der hier genannten Komponenten enthalten kann.

Hierbei ist zu beachten, dass die Komponenten teilweise neben ihrer Wirkung als Lösemittel auch selbst eine antimikrobielle Wirkung aufweisen, beispielsweise Ethanol. In diesem Falle sind sie einsetzbaren Mengen der Systeme (a) und (b) nicht kombinierbar, vielmehr ist beispielsweise der Einsatz von Ethanol auf den angegebenen Mengenbereich des Lösemittelsystems beschränkt, auch wenn hier eine zusätzliche Wirkung als antimikrobielles Mittel erzielt wird. Auch wenn Ethanol als Lösemittel eingesetzt wird, wird wenigstens ein zusätzlicher antimikrobieller Wirkstoff aus der oben beschriebenen Gruppe (a) eingesetzt.

In einer bevorzugten Ausführungsform enthält die Zusammensetzung als einziges Lösemittel Isopentan. In einer weiteren Ausführungsform enthält die Zusammensetzung als einziges Lösemittel Ethanol. In der vorliegenden Zusammensetzung bewirkt der Einsatz von Ethanol nicht wie in kosmetischen Zusammensetzungen auf Alkoholbasis eine Austrocknung der Haut, weil die Zusammensetzung außer dem Lösemittelsystem einen hohen Lipid- und / oder Ölanteil aufweist.

### Kosmetische Hilfs- und Zusatzstoffe (c):

Die kosmetische Zusammensetzung kann in kosmetischen Mitteln übliche Hilfs- und Zusatzstoffe enthalten, soweit sie in den Phasen (b) und / oder (d) löslich oder mit diesen mischbar sind. Als übliche Zusatzstoffe werden hier insbesondere Parfüms, bevorzugt Parfümöle, ätherische Öle oder Duftöle, aber auch Essenzen betrachtet. Dem Einsatz dieser Öle/Essenzen ist lediglich in Bezug auf die gewünschte Duftnote eine Grenze gesetzt, so dass jedes auf dem Markt befindliche Öl oder jede Essenz dieser Art in die erfindungsgemäße Zusammensetzung eingearbeitet werden kann.

Darüber hinaus können mit einem Gehalt von insgesamt 0-10 Gew% beispielsweise Vitamine und deren Derivate, z.B. Vitamin E Acetat und Extrakte aus Pflanzen und / oder Früchten sowie UV-Filter eingearbeitet werden.

### Öl-/ Lipidphase (d):

In der Öl- / Lipidphase (d) der kosmetischen Zusammensetzung können Öle oder Lipide oder Öle und Lipide eingesetzt werden. Hierbei kann als Ölphase ein einzelnes Öl oder ein Gemisch von Ölen verwendet werden, als Lipidphase kann ein einzelner Lipidtyp oder ein Gemisch verschiedener Lipidtypen eingesetzt werden.

Die Ölphase der erfindungsgemäßen Formulierungen kann gewählt sein aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 30, bevorzugt 8 bis 24, insbesondere 12 bis 18 C-Atomen. Die Fettsäuretriglyceride können beispielsweise ausgewählt werden aus der Gruppe der synthetischen, halbsynthetischen und natürlichen pflanzlichen Öle.

Als pflanzliche Öle sind beispielsweise geeignet: Aloe vera Öl, Aprikosenkernöl, Avocadoöl, Baobaböl, Bienenwachs, Calendulaöl, Distelöl, Erdnussöl, Hanföl, Jojobaöl, Kamillenöl, Kokosnussfett, Kokosöl, Kürbiskernöl, Leinöl, Macadamiaöl, Maisöl, Mandelöl (süss), Mohnöl, Nachtkerzenöl, Olivenöl, Palmkernöl, Palmöl, Pfirsichkernöl, Rapsöl, Sanddornöl, Sesamöl, Sheabutter, Sojaöl, Sonnenblumenöl, Traubenkernöl, Walnussöl, Weizenkeimöl, Wildrosenöl und ähnliche. Bevorzugt enthält die Zusammensetzung süßes Mandelöl, Nachtkerzenöl, Olivenöl oder Jojobaöl, besonders bevorzugt Mandelöl oder Nachtkerzenöl.

Weitere polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyl-oleat, Isooctylstearat, Isononylstearat, Isononylisononanoat, 2-Ethylhexylpalmitat, 2-Ethylhexyllaurat, 2-Hexyldecylstearat, 2-Octyidodecylpalmitat, Stearylheptanoat, Oleyloleat, Oleylerucat, Erucyloleat, Erucylerucat, Tridecylstearat, Tridecyltrimeilitat, sowie synthetische, halbsynthetische und natürliche Gemische solcher Ester, wie z. B. das oben bereits genannte Jojobaöl.

Ferner kann die Ölphase vorteilhaft gewählt werden aus der Gruppe der Dialkylether und Dialkylcarbonate, vorteilhaft sind z. B. Dicaprylylether (Cetiol OE) und/oder Dicaprylylcarbonat, beispielsweise das unter der Handelsbezeichnung Cetiol CC bei der Fa. Cognis erhältliche Öl.

Es ist außerdem möglich die Ölkomponente(n) aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cis-13-Alkyllactat, Di-Cis-is-Aikyitartrat, Triisostearin, Dipentaerythrityl Hexacaprylat/Hexacaprat, Propylenglykolmonoisostearat, Tricaprylin, Dimethylisosorbid zu wählen. Auch beliebige Abmischungen solcher Öl- und Wachskomponenten können im Sinne der vorliegenden Erfindung eingesetzt werden.

Ferner kann die Ölphase auch unpolare Öle enthalten, beispielsweise solche aus der Gruppe der verzweigten und unverzweigten Kohlenwasserstoffe, insbesondere Mineralöl, Vaseline (Petrolatum), Paraffinöl, Squalan und Squalen, Polyolefine, hydrogenierte Polyisobutene und Isohexadecan, sowie polymerisierte Silicone (z.B. Belsil^{™} von der Firma Wacker Silicones) Unter den Polyolefinen sind Polydecene die bevorzugten Substanzen.

### Lipide:

Die Öl-/ Lipidphase (d) kann neben oder anstelle der oben genannten Ölkomponenten auch wenigstens ein Lipid oder zwei, drei oder mehrere Lipide umfassen oder aus diesen bestehen. Hierbei können die Lipide ausgewählt sein aus der Gruppe der natürlichen und/oder synthetischen Lipide, der Fettsäuren, der verzweigten und unverzweigten langkettigen Alkohole (Fettalkohole) mit C₈ bis C₃₀, Ester von Fettsäuren mit Alkoholen oder von Fettalkoholen mit Säuren oder der flüssigen Wachse.

### Fettsäuren:

Hexansäure, Heptansäure, Octansäure, Nonansäure, Decansäure, Dodecansäure, Tetradecansäure, Hexadecansäure, Heptadecansäure, Octadecansäure, Eicosan-/Icosansäure, Docosansäure, Tetracosansäure, Hexacosansäure, Octaacosansäure, Triacontansäure. Die entsprechenden Trivialnamen lauten Capronsäure, Önanthsäure, Caprylsäure, Pelargonsäure, Caprinsäure, Laurinsäure, Myristinsäure, Palmitinsäure, Margarinsäure, Stearinsäure, Arachinsäure, Behensäure, Lignocerinsäure, Cerotinsäure, Montansäure, Melissinsäure. Ebenfalls geeignet sind Undecylensäure, Myristoleinsäure, Palmitoleinsäure, Petroselinsäure, Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Eleostearinsäure, Arachidonsäure, Timnodonsäure, Clupanodonsäure, Cervonsäure, Vernolsäure und Rizinolsäure.

### Fettalkohole:

Geeignete Fettalkohole sind beispielsweise gesättigte oder ungesättigte Fettalkohole mit 8 bis 30 Kohlenstoffatomen, wie Octanol, Decanol, Dodecanol (Laurylalkohol), Tetradecanol (Myristylalkohol), Hexadecanol(Cetylalkohol), Heptadecanol, Octadecanol (Stearylalkohol), Octyldodecanol, Eicosanol, Behenylalkohol, n-Docosanol, oder Delta-9-cis-Hexadecenol, Delta-9-Octadecenol, trans-Delta-9-Octadecenol, cis-Delta-11-Octadecenol oder Octadecatrienol.

### Ester von Fettsäuren mit Alkoholen oder von Fettalkoholen mit Säuren:

Geeignete Ester sind solche aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Ester können gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, Glycerylmonostearat, Isostearyl-isostearat, Glyceryltriacetate, Propylenglycol Monolaurat, Menthyllactat, Isostearylpalmitat, Myristylmyristat, C12-15 Alkylbenzoat; Benzylbenzoat, Diisooctyladipat, 2-Octyldodecanol, Propylenecarbonat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester oder flüssigen Wachse.

Des weiteren können Ester einer verzweigten oder unverzweigten aliphatischen C₃ bis C₁₀ Säure mit einem verzweigten oder unverzweigten aliphatischen C₁₆ bis C₂₄ Alkohol in die Lipidphase der Zusammensetzung eingesetzt werden. Bevorzugt sind hierbei Ester der Heptansäure oder der Octansäure mit Stearylalkohol, also Stearylheptanoat oder Stearyloctanoat.

Die Öl-/ Lipidphase (d) enthält bevorzugt wenigstens eine Komponente aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-Triglycerid, Dicaprylylether, Dicaprylyl Carbonat, Butylen Gylcol und Dicaprylat/Dicaprat, Isopropylmyristat und Isopropylpalmitat.

Besonders vorteilhaft sind Mischungen aus C12-15-Alkylbenzoat und Isopropylmyristat, Mischungen aus C12-15-Alkylbenzoat und Isoproyplpalmitat sowie Mischungen aus C12-15-Alkylbenzoat, Isopropylmyristat und Isopropylpalmitat.

Ebenfalls besonders bevorzugt ist eine Mischung aus wenigstens einer Öl- und wenigstens einer Lipidkomponente, beispielsweise aus einem pflanzlichen Öl und C12-15 Alkylbenzoat und ggf. Capryl/Capric Triglycerin, oder aus einem pflanzlichen Öl, C12-15 Alkylbenzoat, Octyldodecanol und ggf. Capryl/Capric Triglycerin.

Die Öl- /Lipidphase (d) wird in einer Menge in die erfindungsgemäße Zusammensetzung eingesetzt, die der Restmenge ad. 100 Gew% der Zusammensetzung entspricht. Dies bedeutet, dass die Phasen (a), (b) und (c) die oben angegebenen Gew% der Zusammensetzung ausmachen und der verbleibende Rest der Zusammensetzung durch die Phase (d) dargestellt wird.

Da es sich bei der erfindungsgemäßen Zusammensetzung um ein homogenes Gemisch von organischen Phasen handelt, in denen gegebenenfalls organische Substanzen gelöst vorliegen, ist es nicht notwendig Emulgatoren zu verwenden und erfindungsgemäß auch bevorzugt, dass keine Emulgatoren in die Zusammensetzung eingearbeitet werden. Insbesondere enthält die Zusammensetzung kein Wasser bzw. keine wässrige Lösung.

Darüber hinaus ist es bevorzugt in das Gemisch keine Antitranspiranzien einzuarbeiten, insbesondere keine antitranspiranten Salze, um die Bildung weißer Flecke auf Haut und Kleidung zu vermeiden. Die Zusammensetzung enthält kein Aluminiumsalz wie Aluminiumchlorhydrat.

Die Phasen (b) und (d), sowie die optionale Phase (c) können nahezu vollständig aus organischen, insbesondere öligen oder lipidhaltigen Komponenten bestehen. In diesem Zusammenhang bedeutet "nahezu vollständig", dass diese Phasen zu mehr als 90%, bevorzugt mehr als 95%, weiter bevorzugt zu mehr als 98% und insbesondere bevorzugt zu wenigstens 99% aus organischen, insbesondere öligen oder lipidhaltigen Komponenten bestehen, wobei in diesem Falle Isopentan den öligen Komponenten zuzuordnen ist. Darüber hinaus stellt die Phase (a) der Zusammensetzung eine in den Phasen (b) und/oder (d) vollständig lösliche oder homogen mit diesen Phasen mischbare Phase dar. Somit kann die erfindungsgemäße Zusammensetzung insgesamt nahezu vollständig aus organischen, insbesondere aus öligen oder lipidhaltigen Komponenten bestehen. Der hohe Anteil der öligen und oder lipidhaltigen Phasen trägt zur hohen Pflegewirkung der Zusammensetzung der Haut bei, so dass ein Austrocknen der Haut auch bei regelmäßiger Anwendung vermieden wird.

Die kosmetische Zusammensetzung wird ein durch Gas ausgetriebenes Spray aus einem unter Druck stehenden Behälter ("Sprayformulierung") bereitgestellt.
Bei der Bereitstellung der Sprayformulierung kann die Zusammensetzung entweder ohne weitere Zusätze in einen Behälter gefüllt werden, der unter Druck stabil bleibt, und anschließend wird dem Behälter ein Gas zugesetzt, das das Austreiben der Formulierung aus dem Behälter zu einem späteren Zeitpunkt ermöglicht, oder die Zusammensetzung wird mit einem Gas oder einer Gasmischung versetzt, bevor sie in einen geeigneten Behälter gefüllt wird. Hierbei ist der Zusatz des Gases in die Zusammensetzung bevorzugt.

Die Zusammensetzung kann beispielsweise mit einem komprimierten oder unter Druck verflüssigten Gas der Gruppe Propan, n-Butan, iso-Butan oder Dimethylether (DME) versetzt werden, bevorzugt mit einem Gemisch aus n-Butan und Propan. Diese Gase, insbesondere die beiden letztgenannten Gase können in sämtlichen / beliebigen Mischungsverhältnissen eingesetzt werden.

Ein solches Gasgemisch kann beispielsweise unter Druck vollständig in die Zusammensetzung eingearbeitet werden. Das gesamte Gemisch, also die kosmetische Zusammensetzung einschließlich des Gases liegt als homogene flüssige Phase und Dampfdruckphase des Treibmittels vor.

Der Anteil der Gasphase an dem so hergestellten Gemisch kann bis zur Gassättigung reichen, bevorzugt wird eine Gaskonzentration von bis zu 90,0% eingestellt.

Da sich alle Bestandteile der kosmetischen Zusammensetzung ineinander lösen oder homogen miteinander mischbar sind, ist die Herstellungsweise der kosmetischen Zusammensetzung denkbar einfach. Die einzelnen Komponenten müssen lediglich zusammengegeben werden und miteinander vermischt werden, ohne dass eine Dispersion oder Emulsion der Bestandteile notwendig wird. Somit ist auch keine aufwendige Ausstattung mit Maschinen für die Herstellung der erfindungsgemäßen Produktes notwendig, da das Produkt weder als Emulsion, noch als Suspension oder Dispersion hergestellt wird. Das Produkt stellt vielmehr eine stabile Lösung der Komponenten dar, woraus sich eine einphasige klare bis opaleszente Lösung ergibt, die auch nicht vor Gebrauch als Körperpflegemittel geschüttelt zu werden braucht.

Die erfindungsgemäße kosmetische Zusammensetzung kann in erster Linie zur Verringerung der Entwicklung eines unangenehmen Körpergeruches eingesetzt werden. Bevorzugt wird die Zusammensetzung als Deodorant, Körperspray, Körper- oder Gesichtspflegemittel oder Make-up Entfernungsmittel verwendet.

## Patentansprüche

1. Sprayformulierung, bestehend aus einem Gemisch einer kosmetischen Zusammensetzung und einem Gas oder Gasgemisch als Treibmittel, wobei das Gemisch aus kosmetischer Zusammensetzung und Gas als homogene flüssige Phase und Dampfdruckphase des Treibmittels vorliegt und die kosmetische Zusammensetzung
(a) 20 - 25 Gew% ein antimikrobielles Wirksystem, umfassend mindestens einen Wirkstoff ausgewählt aus der Gruppe, enthaltend 2,4,4'-Trichlor-2'-hydroxydiphenylether (Triclosan), Phenoxyethanol, Ethylhexylglycerin, Parabenverbindungen, Isothiazoline und in organischen Lösungsmitteln lösliche Zinkverbindungen,
(b) 0 - 70 Gew% ein Lösungsmittelsystem für (a) und/oder (c), und/oder (d), wobei das Lösungsmittelsystem wenigstens ein Lösungsmittel, ausgewählt aus der Gruppe der kurzkettigen Alkohole mit 2 bis 6 Kohlenstoffatomen, der kurzkettigen Kohlenwasserstoffe mit 5 oder 6 Kohlenstoffatomen, der Glykole oder der Glykolether umfasst, wobei der Anteil an Ethanol auf maximal 40 Gew% beschränkt ist, sofern Ethanol als Lösungmittel verwendet wird,
(c) optional einen oder mehrere übliche Hilfs- oder Zusatzstoffe, soweit sie in (a), (b) und/ oder (c) löslich oder mit diesen mischbar sind, und
(d) ad 100% ein Öl-/ Lipidsystem
umfasst, wobei die Zusammensetzung kein Aluminiumsalz enthält.

2. Sprayformulierung gemäß Anspruch 1, **dadurch gekennzeichnet, dass** das Lösungsmittelsystem wenigstens ein Lösungsmittel, ausgewählt aus der Gruppe, enthaltend Ethanol, Propanol, n-Butanol, Isobutanol, n-Pentanol, Isopentanol,n-Hexanol, Isohexanol, n-Pentan, Isopentan, n-Hexan, Isohexan, Monoprophylenglykol, Dipropylenglykol, Dipropylenglykolmonomethylether und 3-Butoxypropan-2-ol umfasst.

3. Sprayformulierung gemäß Anspruch 1 oder 2, **dadurch gekennzeichnet, dass** der/die Hilfs- und Zusatzstoffe (c) ausgewählt ist/sind aus der Gruppe bestehend aus Parfüm, Parfümöl, ätherischem Öl, Duftöl, Vitamine und deren Derivate, Extrakte aus Pflanzen und Früchten, UV-Filter.

4. Sprayformulierung gemäß einem der Ansprüche 1 bis 3, **dadurch gekennzeichnet, dass** das Öl-/ Lipidsystem (d) wenigstens eine Komponente umfasst, ausgewählt aus der Gruppe der Lipide, Triglyceride, Fettsäuren, verzweigten und unverzweigten langkettigen Alkohole mit C₈ bis C₃₀, Ester von Fettsäuren mit Alkoholen oder von Fettalkoholen mit Säuren, pflanzlichen Öle, Paraffinöl, Lanolin und Sheabutter.

5. Sprayformulierung gemäß einem der Ansprüche 1 bis 4, **dadurch gekennzeichnet, dass** die Zusammensetzung mit einem komprimierten oder unter Druck verflüssigten Gas der Gruppe Propan, n-Butan, iso-Butan, Dimethylether (DME) oder Gemischen derselben versetzt ist, bevorzugt mit einem Gemisch aus n-Butan und Propan.

6. Sprayformulierung gemäß einem der Ansprüche 1 bis 5, **dadurch gekennzeichnet, dass** die Zusammensetzung keine(n) Emulgator(en) enthält.

7. Sprayformulierung gemäß einem der Ansprüche 1 bis 6, **dadurch gekennzeichnet, dass** die Zusammensetzung keinen schweißhemmenden Wirkstoff (Antiperspirant) enthält.

8. Sprayformulierung gemäß einem der Ansprüche 1 bis 7, **dadurch gekennzeichnet, dass** die kosmetische Zusammensetzung eine klare bis opaleszente Lösung darstellt.

9. Verwendung einer Sprayformulierung gemäß einem der Ansprüche 1 bis 8 als Deodorant, Körperspray, Körper- oder Gesichtspflegemittel oder Make-up Entfernungsmittel.

## Claims

1. Spray formulation, consisting of a mixture of a cosmetic composition and a gas or gas mixture as propellant, wherein the mixture of cosmetic composition and gas is present as homogeneous liquid phase and vapour pressure phase of the propellant, and the cosmetic composition comprises
(a) 20-25 wt% of an antimicrobial active system, comprising at least one active ingredient selected from the group consisting of 2,4,4'-trichloro-2'-hydroxydiphenyl ether (triclosan), phenoxyethanol, ethylhexylglycerol, paraben compounds, isothiazolines, and zinc compounds that are soluble in organic solvents,
(b) 0-70 wt% of a solvent system for (a) and/or (c), and/or (d), wherein the solvent system comprises at least one solvent selected from the group of the short-chain alcohols having 2 to 6 carbon atoms, the short-chain hydrocarbons having 5 or 6 carbon atoms, the glycols or the glycol ethers, wherein the proportion of ethanol is limited to at most 40 wt% if ethanol is used as solvent,
(c) optionally one or more customary auxiliaries or additives, as long as they are soluble in, or miscible with, (a), (b) and/or (c), and
(d) the remainder to 100% of an oil/lipid system,
wherein the composition does not contain any aluminium salt.

2. Spray formulation according to Claim 1, **characterized in that** the solvent system comprises at least one solvent selected from the group consisting of ethanol, propanol, n-butanol, isobutanol, n-pentanol, isopentanol, n-hexanol, isohexanol, n-pentane, isopentane, n-hexane, isohexane, monopropylene glycol, dipropylene glycol, dipropylene glycol monomethyl ether, and 3-butoxypropan-2-ol.

3. Spray formulation according to Claim 1 or 2, **characterized in that** the auxiliary or auxiliaries and additive(s) (c) is/are selected from the group consisting of perfume, perfume oil, essential oil, fragrance oil, vitamins and derivatives thereof, extracts from plants and fruit, and UV filters.

4. Spray formulation according to one of Claims 1 to 3, **characterized in that** the oil/lipid system (d) comprises at least one component selected from the group of lipids, triglycerides, fatty acids, branched and unbranched long-chain C₈-C₃₀ alcohols, esters of fatty acids with alcohols or of fatty alcohols with acids, vegetable oils, paraffin oil, lanolin and shea butter.

5. Spray formulation according to one of Claims 1 to 4, **characterized in that** a compressed gas or gas liquefied under pressure, from the group of propane, n-butane, isobutane, dimethyl ether (DME) or mixtures of same, preferably a mixture of n-butane and propane, has been added to the composition.

6. Spray formulation according to one of Claims 1 to 5, **characterized in that** the composition does not contain any emulsifier(s).

7. Spray formulation according to one of Claims 1 to 6, **characterized in that** the composition does not contain any antiperspirant.

8. Spray formulation according to one of Claims 1 to 7, **characterized in that** the cosmetic composition is a clear to opalescent solution.

9. Use of a spray formulation according to one of Claims 1 to 8 as deodorant, body spray, personal care or facial care product or make-up remover.

## Revendications

1. Formulation d'aérosol, constituée d'un mélange d'une composition cosmétique et d'un gaz ou d'un mélange de gaz en tant qu'agent propulseur, dans laquelle le mélange de composition cosmé-tique et de gaz se présente à l'état de phase homogène liquide et de phase sous pression de vapeur de l'agent propulseur, et la composition cosmétique comprend :
a) de 20 à 25 % en poids d'un système actif antimicrobien, comprenant au moins une substance active choisie dans l'ensemble comprenant les 2,4,4'-trichloro-2'-hydroxy-diphényl-éther (triclosan), phénoxy-éthanol, éthylhexyl-glycérine, composés de type parabène, isothia-zoline, et composés du zinc solubles dans des solvants organiques,
b) de 0 à 70 % en poids d'un système solvant du ou des composant(s) (a) et/ou (c) et/ou (d), lequel système solvant comprend au moins un solvant choisi dans l'ensemble formé par les alcools à chaîne courte comportant de 2 à 6 atomes de carbone, les hydrocarbures à chaîne courte comportant 5 ou 6 atomes de carbone, les glycols et les éthers de glycol, étant entendu que si on utilise de l'éthanol comme solvant, la proportion d'éthanol est limitée à un maximum de 40 % en poids,
c) en option, un ou plusieurs adjuvants ou additifs auxiliaires usuels, pourvu qu'ils soient solubles dans, ou miscibles avec, le ou les composant(s) (a), (b) et/ou (c),
d) et un système huile/lipide en complément à 100 %,
étant entendu que la composition ne contient aucun sel d'aluminium.

2. Formulation d'aérosol conforme à la revendication 1, **caractérisée en ce que** le système solvant comprend au moins un solvant qui est choisi dans l'ensemble formé par les suivants : éthanol, propanol, n-butanol, isobutanol, n-pentanol, isopentanol, n-hexanol, isohexanol, n-pentane, isopentane, n-hexane, isohexane, monopropylèneglycol, dipropylèneglycol, éther monométhylique de dipropylèneglycol, et 3-butoxy-propan-2-ol.

3. Formulation d'aérosol conforme à la revendication 1 ou 2, **caractérisée en ce que** le ou les adjuvant(s) ou additif(s) auxiliaire(s) (c) est ou sont choisi(s) dans l'ensemble formé par les parfums, huiles parfumées, huiles essentielles, huiles odorantes, vitamines et leurs dérivés, extraits de plantes ou de fruits, et filtres UV.

4. Formulation d'aérosol conforme à l'une des revendications 1 à 3, **caractérisée en ce que** le système huile/lipide (d) comprend au moins un composant choisi dans l'ensemble formé par les lipides, triglycérides, acides gras, alcools à chaîne longue, en C₈ à C₃₀, ramifiés ou non ramifiés, esters d'acides gras et d'alcools ou d'alcools gras et d'acides, huiles végétales, huile de paraffine, lanoline et beurre de karité.

5. Formulation d'aérosol conforme à l'une des revendications 1 à 4, **caractérisée en ce qu'**à la composition est ajouté un gaz comprimé ou un gaz liquéfié sous pression, choisi dans l'ensemble formé par les propane, n-butane, isobutane, diméthyl-éther (DME) et leurs mélanges, et de préférence un mélange de n-butane et de propane.

6. Formulation d'aérosol conforme à l'une des revendications 1 à 5, **caractérisée en ce que** la composition ne contient pas d'émulsifiant(s).

7. Formulation d'aérosol conforme à l'une des revendications 1 à 6, **caractérisée en ce que** la composition ne contient pas d'agent anti-transpiration.

8. Formulation d'aérosol conforme à l'une des revendications 1 à 7, **caractérisée en ce que** la composition cosmétique se présente sous forme d'une solution limpide à opalescente.

9. Utilisation d'une formulation d'aérosol conforme à l'une des revendications 1 à 8 en tant que déodorant, spray corporel, produit pour soins du corps ou du visage, ou produit pour démaquillage.
